# EUROPEAN PATENT SPECIFICATION

(11) **EP 3 272 882 B1**
(45) Date of publication and mention of the grant of the patent: **20.02.2019**
(21) Application number: 17189590.7
(22) Date of filing: 21.09.2012
(51) Int. Cl.: C12Q 1/68

(54) **MATERIALS AND METHODS FOR PROGNOSIS OF PROGRESSION OF BARRETT'S ESOPHAGUS**
MATERIALIEN UND VERFAHREN ZUR VORHERSAGE DER ENTWICKLUNG VON BARRETT-ÖSOPHAGUS
MATÉRIAUX ET PROCÉDÉS POUR LE PRONOSTIC DE LA PROGRESSION DE L' SOPHAGE DE BARRETT

(30) Priority: 23.09.2011 US 201161538291 P
(43) Date of publication of application: 24.01.2018
(62) Divisional of application: 12766631.1
(73) Proprietor: Academisch Medisch Centrum, 1105 AZ Amsterdam (NL)
(72) Inventor: KRISHNADATH, Kausilia Krishnawatie, 2051 EL Overveen (NL)
(74) Representative: Modiano, Micaela Nadia

(56) References cited:
- WO-A1-2009/049129
- WO-A2-2006/089163
- RYGIEL AGNIESZKA M ET AL: "Efficient automated assessment of genetic abnormalities detected by fluorescence in situ hybridization on brush cytology in a Barrett esophagus surveillance population.", CANCER 15 MAY 2007, vol. 109, no. 10, 15 May 2007 (2007-05-15) , pages 1980-1988, XP002687128, ISSN: 0008-543X
- BRANKLEY SHANNON M ET AL: "The development of a fluorescence in situ hybridization assay for the detection of dysplasia and adenocarcinoma in Barrett's esophagus.", THE JOURNAL OF MOLECULAR DIAGNOSTICS : JMD MAY 2006, vol. 8, no. 2, May 2006 (2006-05), pages 260-267, XP002687129, ISSN: 1525-1578
- REID B J ET AL: "Predictors of progression in Barrett's esophagus II: baseline 17p (p53) loss of heterozygosity identifies a patient subset at increased risk for neoplastic progression.", THE AMERICAN JOURNAL OF GASTROENTEROLOGY OCT 2001, vol. 96, no. 10, October 2001 (2001-10), pages 2839-2848, XP002687130, ISSN: 0002-9270
- PRASAD G A ET AL: "Utility of Biomarkers in Prediction of Response to Ablative Therapy in Barrett's Esophagus", GASTROENTEROLOGY, ELSEVIER, PHILADELPHIA, PA, vol. 135, no. 2, 1 August 2008 (2008-08-01), pages 370-379, XP023901344, ISSN: 0016-5085, DOI: 10.1053/J.GASTRO.2008.04.036 [retrieved on 2008-05-07]
- DOLAN KEVIN ET AL: "Loss of heterozygosity on chromosome 17p predicts neoplastic progression in Barrett's esophagus.", JOURNAL OF GASTROENTEROLOGY AND HEPATOLOGY JUN 2003, vol. 18, no. 6, June 2003 (2003-06), pages 683-689, XP002687131, ISSN: 0815-9319
- FAHMY MONA ET AL: "Chromosomal gains and genomic loss of p53 and p16 genes in Barrett's esophagus detected by fluorescence in situ hybridization of cytology specimens", MODERN PATHOLOGY, NATURE PUBLISHING GROUP, US, vol. 17, no. 5, 1 May 2004 (2004-05-01), pages 588-596, XP002574692, ISSN: 0893-3952, DOI: 10.1038/MODPATHOL.3800088 [retrieved on 2004-03-12]
- PRASAD GANAPATHY A ET AL: "Predictors of progression in Barrett's esophagus: current knowledge and future directions.", THE AMERICAN JOURNAL OF GASTROENTEROLOGY JUL 2010, vol. 105, no. 7, July 2010 (2010-07), pages 1490-1502, XP002687132, ISSN: 1572-0241
- MALEY CARLO C ET AL: "Selectively advantageous mutations and hitchhikers in neoplasms: p16 lesions are selected in Barrett's esophagus.", CANCER RESEARCH 15 MAY 2004, vol. 64, no. 10, 15 May 2004 (2004-05-15), pages 3414-3427, XP002687133, ISSN: 0008-5472
- WONG D J ET AL: "p16(INK4a) lesions are common, early abnormalities that undergo clonal expansion in Barrett's metaplastic epithelium.", CANCER RESEARCH 15 NOV 2001, vol. 61, no. 22, 15 November 2001 (2001-11-15), pages 8284-8289, XP002687134, ISSN: 0008-5472
- A. M. RYGIEL ET AL: "Gains and Amplifications of c-myc, EGFR, and 20.q13 Loci in the No Dysplasia-Dysplasia-Adenocarcinoma Sequence of Barrett's Esophagus", CANCER EPIDEMIOLOGY, BIOMARKERS & PREVENTION, vol. 17, no. 6, 1 June 2008 (2008-06-01), pages 1380-1385, XP055043894, ISSN: 1055-9965, DOI: 10.1158/1055-9965.EPI-07-2734
- POWELL E L ET AL: "Concordant loss of MTAP and p16/CDKN2A expression in gastroesophageal carcinogenesis - Evidence of homozygous deletion in esophageal noninvasive precursor lesions and therapeutic implications", AMERICAN JOURNAL OF SURGICAL PATHOLOGY, RAVEN PRESS, NEW YORK, NY, US, vol. 29, no. 11, 1 November 2005 (2005-11-01), pages 1497-1504, XP008116889, ISSN: 0147-5185, DOI: 10.1097/01.PAS.0000170349.47680.E8
- P C GALIPEAU ET AL: "Clonal expansion and loss of heterozygosity at chromosome 9p and 17p in premalignant esophageal (Barrett's) tissue", J. NAT. CANCER INST., vol. 91, no. 24, 15 December 1999 (1999-12-15), pages 2087-2095, XP55082745, DOI: 10.1093/jnci/91.24.2087

## Description

### TECHNICAL FIELD

The present disclosure relates to the prognosis of progression of Barrett's esophagus, the determination of genetic abnormalities, and *in situ* hybridization, as well as a set of probes and a kit useful for prognosis of Barrett's esophagus.

### BACKGROUND

Barrett's esophagus is a metaplastic, pre-malignant condition in which normal squamous epithelium of the distal esophagus is replaced by columnar mucosa, which is similar to the lining of the intestine. This process is called intestinal metaplasia. The condition is named for Norman Rupert Barrett, a surgeon who drew attention to the "short esophagus" in a report that he published in 1950 (Barrett, Br. J. Surg. 38: 175-182 (1950); see, also, review by Shepherd, Esophagus 1: 17-29 (2003)).

Little is known about the molecular events that effect the transformation of the esophageal squamous epithelium. The FHIT gene, which spans the common fragile site FRA3B, reportedly is frequently deleted in Barrett's metaplasia and esophageal carcinomas (Michael et al., Oncogene 15(14): 1653-1659 (Oct. 1997)). DNA gains on chromosomes 2q, 3q, 7p, 17q, and 22q have been observed in metastatic primary Barrett's adenocarcinoma (Walch et al., Mod. Pathol. 13(7): 814-824 (2000)). HER-2/*neu* amplification reportedly has been associated with strong mRNA over-expression and strong membranous HER-2/*neu* immunostaining in Barrett's adenocarcinoma and high-grade dysplasia (Walch et al., Lab. Invest. 81: 791-801 (2001)). HER-2/*neu* gene amplification reportedly predicts poor survival in Barrett's esophagus-associated adenocarcinoma (Brien et al., Human Pathol. 31(1): 35-39 (January 2000)). Recent studies suggest a pathogenic role for the *Cdx* genes, which belong to the homeobox gene family of transcription factors and are known to mediate the differentiation of intestinal epithelial cells. The *Cdx2* gene is expressed by epithelial cells in the small intestine and the large intestine but normally not in the esophagus and the stomach. Silberg et al. (Gastroenterology 122: 689-696 (2002)) reportedly found that they could induce intestinal metaplasia in the stomachs of mice by forcing the gastric epithelial cells to express *Cdx2.* Immunostaining for *Cdx2* has been found in 100% of biopsy specimens of Barrett's specialized intestinal metaplasia (Phillips et al., Am. J. Surg. Pathol. 27: 1442-1447 (2003)). p53 locus loss and/or aneusomy of chromosomes 6, 7, 11, and 12 was/were reportedly detected in 95% of cases positive for dysplasia/carcinoma by biopsy, whereas loss of the p16 locus reportedly was commonly seen in patients with and without dysplasia/carcinoma (Fahmy et al., Modern Pathol. 17: 588-596 (2004)). DNA sequence copy number gains reportedly were detected for *SNRPN*, *GNLY, NME1*, *DDX15*, *ABCB1* (*MDR*), *ATM, LAMA3, MYBL2, ZNF217, TNFRSF6B, MSH2, TERC, SERPINE1, AFM137XA11, IGF1R,* and *PTPN1,* whereas DNA sequence copy number losses reportedly were detected for *PDGFB, D17S125, AKT3, RASSFI, FHIT, CDKN2A* (p16), and *SAS* (*CDK4*) in Barrett's adenocarcinoma (Albrecht et al., The J. of Pathol. 203(3): 780-788 (July 2004)). Amplification of chromosome 4 reportedly was observed in Barrett's tissues (Doak et al., Experimental Molec. Pathol. 77(1): 26-33 (Aug. 2004)). Cyclin D1 over-expression, C-erbB2(+), high plasma adenomatous polyposis coli levels, cyclooxygenase-2 over-expression, and increased E-cadherin staining were identified as biomarkers for risk stratification for patients with Barrett's esophagus (Kyrgidis et al., J. Surgical Research 125(2): 189-212 (May 2005)). p53 amplification reportedly confirmed multifocal dysplasia in all cases tested, whereas the loss of chromosome Y reportedly may be a protective marker of high-grade dysplasia in male patients (Cestari et al., Cancer Letters 251(2): 2278-287 (June 2007)). p53 amplification reportedly was observed in adenocarcinomas *in situ* and intramucosal adenocarcinomas (Ooi et al., Pathol. Int'1 60(6): 466-471 (June 2010)). p16 loss reportedly can help predict loss of dysplasia in patients with Barrett's esophagus and high-grade dysplasia/mucosal cancer (Prasad et al., Gastroenterology135(2): 379-379 (August 2008)). Single-nucleotide polymorphism arrays reportedly confirmed previously described genomic alterations, e.g., amplification on 8q (*CMYC*) and 20q13 or deletion/loss of heterozygosity on 3p (*FHIT*) and 9p (*CDKN2A*), in esophageal adenocarcinomas (Wiech et al., Lab. Invest. 89: 385-397 (2009)).

Despite the numerous studies on potential biomarkers that have been published, none of the potential biomarkers identified to date has been evaluated in a cross-sectional study of selected groups of patients with Barrett's esophagus. In addition, prospective analyses on large, unselected cohorts lack progression. One of the few prognostic markers, which, in prospective follow-up studies, has been associated with progression of Barrett's esophagus into high-grade dysplasia or esophageal adenocarcinoma is aneuploidy/increased G2M fractions (Reid et al., Gastroenterology 102: 1212-1219 (1992); and Rabinovitch et al., Am. J. Gastroenterol. 96: 3071-3083 (2001)). p53 protein overexpression, gene mutations and p53 loss of heterozygosity (LOH) also has been evaluated with respect to prognosis in Barrett's esophagus (Reid et al., Am J. Gastroenterol. 96: 2839-2848 (2001); and Dolan et al., J. Gastroenterol. Hepatology 18(6): 683-9 (2003). With that said, conventional methodologies for detecting ploidy changes and p53 gene abnormalities are currently not feasible for routine clinical assays.

Data obtained at the Mayo Clinic for patients having endoscopic examinations between 1976 and 1989 suggest that Barrett's esophagus affects about 1% of adults in the United States between the ages of 80 and 89, the average age for developing Barrett's esophagus is 40 years, and the average age at the time of diagnosis is 63 years (Cameron et al., Gastroenterology 103: 1241-1245 (1992)). Men are afflicted more than women, and Caucasian men are affected more than men of other races.

There are no symptoms associated with Barrett's esophagus. However, it is commonly found in people with gastroesophageal reflux disease (GERD) (5-10%; Kyrgidis et al. (May 2005), *supra*). Barrett's esophagus is the most important risk factor and precursor lesion of esophageal adenocarcinoma, which is an aggressive malignancy with an overall five-year survival rate of less than 20%. Early detection and treatment are critical to survival. In view of the foregoing, many physicians recommend that adults, who are older than 40 and have had GERD for a number of years, undergo an upper gastrointestinal (GI) endoscopy and multiple biopsies to check for Barrett's esophagus. Also recommended is that those adults, who have been diagnosed with Barrett's esophagus, undergo periodic upper GI endoscopy and multiple biopsies to detect the appearance of pre-cancerous cells (dysplasia). The British Society of Gastroenterology recommends that adults, who have been diagnosed with Barrett's esophagus but do not have any dysplasia, be screened at least every two years, whereas adults with low-grade dysplasia should be screened every six months, and patients with high-grade dysplasia should undergo treatment and be screened every three months. Similar guidelines have been defined by the American Society of Gastroenterology.

Barrett's esophagus with High grade dysplasia (HGD) or cancer can be treated using endoscopic or surgical techniques. Endoscopic techniques involve the destruction of the Barrett's lining or the removal of the portion of the Barrett's lining with dysplasia. Phytodynamic therapy (PDT), for example, involves injecting a light-sensitizing agent (e.g., Photofrin) into a vein of a patient and, forty eight hours later, passing a laser light through an endoscope to activate the light-sensitizing agent and destroy Barrett's tissue in the esophagus. Endoscopic mucosal resection (EMR) involves either lifting the Barrett's lining and injecting a solution under it or applying suction to the Barrett's lining and cutting it away. The lining is then removed through the endoscope. Surgical removal of most of the esophagus is recommended if a person with Barrett's esophagus has severe dysplasia or cancer and can tolerate a surgical procedure.

While periodic upper GI endoscopy and 4-quadrant biopsies taken every 1-2 cm of an affected esophagus and of as any suspicious lesions are currently recommended for Barrett patient surveillance. And histology results are considered the gold standard for diagnosing esophageal dysplasia and adenocarcinoma, there are many limitations in the guidelines for endoscopy and biopsy as recommended by the Amercian College of Gastroenterology. Such limitations include limited visibility of early lesions, difficult endoscopic recognition of early lesions, limited sampling and sampling errors of the affected area, lengthy, and oftentimes, frequent procedures for biopsy collection, inter- and intra-observer variability, and an inability to predict patient progression from Barrett's esophagus to esophageal carcinoma (Brankley et al., J. Mol. Diagn. 8(2): 260-267 (May 2006)). In this regard, it is important to note that the progression from Barrett's esophagus to esophageal adenocarcinoma is a gradual, lengthy process that may take years, if not decades, and a recent systematic review of over 40 studies on the progression of Barrett's esophagus showed that the overall estimate of cancer incidence in patients with Barrett's esophagus is 0.61%/year. In other words, on a yearly basis more than 99% of patients with Barrett's esophagus will not develop esophageal adenocarcinoma. Thus, not only are current surveillance methods unreliable, they are not cost-effective.

Various additional endoscopic techniques for recognizing early esophageal neoplasia have been proposed. Such techniques include chromoendoscopy (mucosal staining with dyes), endosonography, optical coherence tomography, and spectroscopy using reflectance, absorption, light-scattering, fluorescence, and Raman detection methods (Canto et al. Gastrointest. Endosc. 51: 561-568 (2000); Scotiniotis et al., Gastrointest. Endosc. 54: 689-696 (2001); Kobayashi et al., Gastrointest. Endosc. 47: 515-523 (1998); Georgakoudi et al., Gastroenterology 120: 1620-1629 (2001); Kendall et al., J. Pathol. 200: 602-609 (2003); and Conio et al., Lancet Oncol. 6: 311-321 (2005)).

In addition, various molecular markers for cancer risk have been proposed as alternatives to random biopsy sampling for dysplasia in Barrett's esophagus. Examples include *p53* and cyclin D1 expression (Skacel et al., Am. J. Gastroenterol. 97: 2508-2513 (2002); and Bani-Hani et al., J. Nat'l Cancer Inst. 92: 1316-1321 (2000)). Although preliminary study results are promising, none of these techniques or markers has been shown to provide sufficient clinical information to justify routine use in surveillance.

In view of the foregoing, there remains a need in the art for an objective and reliable method for prognosticating the development of esophageal adenocarcinoma in patients with Barrett's esophagus. The method can be used to stratify patients with Barrett's esophagus into low and high risk groups and subsequently for defining more cost effective surveillance intervals. It is an object of the present disclosure to provide a method, which does not suffer from the disadvantages of currently available methods, and materials for use in the method. This and other objects and advantages, as well as inventive features, will become apparent from the detailed description provided herein.

WO2006089163 discloses a a method for screening for an esophageal carcinoma using a set of probes comprises at least three chromosomal probes selected from the group consisting of an 8q24.12-13 locus-specific probe, a 7p12 locus-specific probe, a 17q11.2-12 locus-specific probe, a 20q13 locus- specific probe, a chromosome enumeration probe for chromosome 9, a chromosome enumeration probe for chromosome 7, a 5q21-22 locus-specific probe, a 5p15 locus-specific probe, a 17p13.1 locus-specific probe, a chromosome enumeration probe for chromosome 17 and a 9p21 locus-specific probe.

### SUMMARY

The present invention provides a method of prognosticating progression of Barrett's esophagus in a patient as defined in the claims, which method comprises:
(a) contacting a sample of esophageal cells obtained from the patient with a set of detectably labeled probes consisting of (i) a probe for p16 which hybridizes to p21 on human chromosome 9, (ii) a probe for p53 which hybridizes to p13 on human chromosome 17, (iii) a centromeric probe for chromosome 7, (iv) a centromeric probe for chromosome 17, and (v) a probe for Her2 which hybridizes to q11 on human chromosome 17 under hybridization conditions, wherein each probe is detectably labeled with a distinct fluorophore,
(b) detecting formation of a hybridization complex between each of the detectably labeled probes and a target nucleic acid sequence by assessing a signal from each of the distinct fluorophores,
(c) determining the copy numbers of the p16 gene, the p53 gene, chromosome 7, chromosome 17, and the Her2 gene by quantifying the signal from each of the distinct fluorophores,
(d) comparing the copy numbers determined in (c) to a pre-determined cut-off for each of the p16 gene, the p53 gene, chromosome 7, chromosome 17, and the Her2 gene, wherein a copy number greater than the pre-determined cut-off indicates a gain of the gene or chromosome and a copy number less than the pre-determined cut-off indicates a loss of the gene or chromosome, and wherein loss of the p16 gene, loss of the p53 gene, chromosome 7 aneuploidy, chromosome 17 aneuploidy, and/or Her2 gene amplification indicates progression of Barrett's esophagus.
The method of the present invention can further comprise detecting in the sample of esophageal cells a gain of chromosome 20q and/or a gain of the c-myc gene.
In the method of the present invention, the sample of esophageal cells can be obtained by a brush cytology method.

A method of prognosticating progression of Barrett's esophagus in a patient is described in the present disclosure. The method comprises detecting in a sample of esophageal cells from the patient at least one abnormality selected from the group consisting of p16 loss, p53 loss, chromosome 7 aneuploidy, and chromosome 17 aneuploidy. The presence of at least one abnormality indicates that Barrett's esophagus will likely progress towards esophageal adenocarcinoma. Preferably, at least one abnormality is p16 loss. More preferably, at least one abnormality is p16 loss, chromosome 7 aneuploidy, and chromosome 17 aneuploidy. The method can further comprise detecting in the sample of esophageal cells from the patient at least one abnormality selected from the group consisting of 20q gain, C-myc gain, and Her2 gain. Preferably, the sample of esophageal cells is obtained by a brush cytology method.

A set of probes is also described in the present disclosure. The set of probes comprises a probe for p16 (loss), a probe for p53 (loss), a probe for chromosome 7 (aneuploidy), and a probe for chromosome 17 (aneuploidy). The set of probes can comprise at least one additional probe. The at least one additional probes can be selected from the group consisting of a probe for 20q (gain), a probe for c-myc (gain), and a probe for Her2 (gain). Thus, the set of probes can comprise a probe for p16 (loss), a probe for p53 (loss), a probe for chromosome 7 (aneuploidy), a probe for chromosome 17 (aneuploidy), a probe for 20q (gain), a probe for c-myc (gain), and a probe for Her2 (gain).

Also described in the present disclosure is a kit. The kit comprises (a) a set of probes that enables prognosis of progression of Barrett's esophagus in a patient and instructions for prognosticating the progression of Barrett's esophagus in a patient. The set of probes comprises a probe for p16, a probe for p53, a probe for chromosome 7, and a probe for chromosome 17. The instructions comprise determining in a sample of esophageal cells from a patient at least one abnormality selected from the group consisting of p16 loss, p53 loss, chromosome 7 aneuploidy, and chromosome 17 aneuploidy. The set of probes can comprise at least one additional probe, and the instructions can comprise further instructions. The at least one additional probe can be selected from the group consisting of a probe for 20q, a probe for C-myc, and a probe for Her2. In one embodiment, the abnormality is p16 loss. In another embodiment, the abnormality is p53. In another embodiment, the abnormality is p16 loss, chromosome 7 gain, and chromosome 17 gain. In another embodiment, the abnormality is p16 loss, p53 loss
and chromosome 7 gain. In another embodiment, the abnormality is p16 loss, p53 loss and chromosome 17 gain. In another embodiment, the abnormality is p53 loss, chromosome 17 gain, and chromosome 7 gain. In another embodiment, the abnormality is p16 loss, p53 loss, chromosome 17 gain, and chromosome 7 gain. In one embodiment, the abnormality is chromosome 7 gain. In another embodiment, the abnormality is chromosome 17 gain. In one embodiment, the abnormality is chromosome 7 gain and chromosome 17 gain. The further instructions can comprise determining in a sample of esophageal cells from a patient at least one additional abnormality selected from the group consisting of 20q gain, C-myc gain, and Her2 gain.

### BRIEF DESCRIPTION OF THE FIGURES

Fig. 1 is a graph of cumulative rate of survival vs. follow-up (months), which shows the use of the Kaplan Meier curve to represent the follow-up data for the progression of patient's with Barrett's esophagus. The top line represents the 'marker-negative' group, whereas the bottom line represents the 'marker-positive' group. Each drop on the bottom line represents a progression in the stage of dysplasia. The difference in progression of the two groups over time is statistically significant (log-rank test, p-value=0.01).
Fig. 2 is a graph of cumulative rate of progression-free survival vs. follow-up (months), which shows the use of the Kaplan Meier curve to represent the follow-up data for the progression of patient's with Barrett's esophagus. The top line represents the 'marker-negative' group, whereas the bottom line represents the 'marker-positive' group. Each drop on the bottom line represents a progression in the stage of dysplasia. The difference in progression of the two groups over time is statistically significant (log-rank test, p-value=0.001).
Fig. 3 is graph of % of BE patients with IM, IND, LGD, HGD, and EAC with aneuploidy (CEP7 and CEP 17), Her2/neu amplification, C-myc abnormalities, and 20q abnormalities, wherein the total number of patients in each group is shown in parentheses ().
Fig. 4 is a graph of % of BE patients with IM/IND, LGD, and HGD with aneuoploidy (CEP7 and CEP17), 20q gain, C-myc gain, and Her2 gain, wherein the total number of patients in each group is shown in parentheses ().
Fig. 5 is a graph of sensitivity vs. 1-specificity (ROC curve).
Fig. 6 is a graph of % of BE patients with loss of p16 (9p21), loss of p53 (17p13.1), and aneuploidy (CEP7 and CEP17 aneusomy) for the study cohort IM and validation cohort IM.
Fig. 7a is a graph of percent progression-free survival vs. months for the group that tested positive (test pos) and the group that tested negative (test neg) for FISH p53 abnormalities.
Fig. 7b is a graph of percent progression-free survival vs. months for the group that tested positive (test pos) and the group that tested negative (test neg) for IHC p53 abnormalities.
Fig. 7c is a graph of percent progression-free survival vs. months for the group that tested positive (test pos) and the group that tested negative (test neg) for FISH+IHC p53 abnormalities.

### DETAILED DESCRIPTION

The present disclosure is predicated, at least in part, on the following. Two informative FISH probe sets were defined based on the evaluation of diverse panels of DNA FISH probes that were used to detect several genetic abnormalities in brush cytology specimens from patients with Barrett's esophagus in preclinical exploratory studies (see Rygiel et al., Cancer 109 (10): 1980-1988 (2007); and Rygiel et al., Cancer Epidemiol. Biomarkers Prev. 17(6): 1380-1385 (2008a)). One probe set, which comprised DNA probes for chromosome 9, chromosome 17, chromosome Y, locus-specific region p16 (9p21), and locus-specific region p53 (17p13.1), was considered to be 'potentially prognostic' for the development of dysplasia (i.e., they were found in low frequency in patients with 'no dysplasia' (ND) and increased in frequency in patients with 'indefinite for dysplasia'/'low-grade dysplasia' (IND/LGD) and 'high-grade dysplasia'/'esophageal adenocarcinoma' (HGD/EAC) (Rygiel et al. (2007), *supra*). The other probe set, which comprised DNA probes for locus-specific region Her2/neu (17q11.2), locus-specific region c-myc (8q24.12-13), locus-specific region EGFR (7p12), and the putative oncogenic region on 20q, was considered to be 'diagnostic' for detecting high-grade dysplasia or esophageal adenocarcinoma (i.e., they were found in high frequency in patients with 'high-grade dysplasia' and/or 'esophageal adenocarcinoma') (Rygiel et al. (2008a), *supra*). The sets were combined to create a 'potentially prognostic/diagnostic' FISH probe set comprising DNA probes specific for chromosome 7, chromosome 17, locus-specific region p53 (17p13.1), locus-specific region p16 (9p21), locus-specific region Her-2 (17p11.2-12), and locus-specific region C-Myc (8q24.12). An automated system, specifically the CytoVision SPOT AX system (Applied Imaging, Newcastle, United Kingdom), was used to score FISH results on cytological specimens and subsequently validated by comparing the automated results with manual scores (98% concordance; κ = 0.49-1; see Rygiel et al. (2007), *supra*). Between 2002 and 2004, the automated FISH assay was applied to evaluate the whole Barrett cohort of the _Academic Medical Center, Amsterdam, the Netherlands (AMC; N=151) for the two chromosomal and four locus-specific probes (Rygiel et al. (2007), *supra*). A long-term prospective follow-up study was conducted and is described herein.

FISH probes that seemed to occur at a higher frequency in patients with Barrett's esophagus and either high-grade dysplasia or esophageal adenocarcinoma were selected for evaluation in the prognosis of the progression from dysplasia to invasive disease or esophageal adenocarcinoma. Cytological specimens from 99 patients (ND = 38; INF/LGD = 25; HGD = 22; and EAC = 16) were evaluated using probes for chromosome 7 and the locus-specific regions Her2/Neu (17q11.2), EGFR (7p12), c-myc (8q24), and 20q13.2. Amplifications of the loci were not seen in ND or IND/LGD patients. At least one locus was amplified in 14% (3/22) of HGD patients and 50% (8/16) of EAC patients (p=0.015). This study confirmed that these loci have a high specificity (100%), but relatively low sensitivity (54%; no false positives but a considerable number of false negatives), for identifying the presence of HGD and/or EAC (Rygiel et al. (2008a), *supra*). Unknown was whether or not these loci were indicative of the short-term progression of HGD to invasive disease or EAC.

In order to improve the sensitivity of the diagnostic probe set, aneuploidy was also evaluated, since it is one of the few markers that has been evaluated with regard to the progression of Barrett's esophagus (Reid et al. (1992), *supra*; and Rabinovitch et al. (2001), *supra*). Ploidy status by FISH was assessed by using specific centromeric probes for chromosomes 7 and 17. Aneuploidy was detected in 15% (6/38) of ND patients, 27% (5/19) IND/LGD patients, 88% (16/18) of HGD patients, and 86% (13/15) of EAC patients, and significantly increased with stage of dysplasia (p<0.001). Aneuploidy determined by FISH for chromosomes 7 and 17 had a high sensitivity (87%) for detecting HGD and EAC and a high specificity (88%).

Combining the probes for the locus-specific regions Her-2 (17q11.2), c-myc (8q24), and 20q13.2 with the probes specific for chromosomes 7 and 17 (indicating ploidy status) yielded a sensitivity of 89% for detecting HGD and/or EAC, while the specificity varied between 87-100% per marker. For comparison, the four probe set consisting of Her-2/Neu (17q11.2), c-myc (8q24), 20q13.2, and p16 (9p21) (Brankley et al., (Brankley,S.M., Wang,K.K., Harwood,A.R., Miller,D.V., Legator,M.S., Lutzke,L.S., Kipp,B.R., Morrison,L.E., and Halling,K.C. (2006). The development of a fluorescence in situ hybridization assay for the detection of dysplasia and adenocarcinoma in Barrett's esophagus. J. Mol. Diagn. 8, 260-267 was also evaluated. Brankley et al. reported that the latter probe set had a sensitivity and specificity, respectively, of 84% and 93% for detecting HGD, and a sensitivity and specificity, respectively, of 94% and 93% for detecting EAC. In the comparison study, the sensitivity and specificity, respectively, of the probe set of Brankley et al. was 88% and between 62% and 100%.

Further analysis of the probe set comprising probes for chromosomes 9, 17, and Y and locus-specific probes for p16 (9p21) and p53 (7p13.1) was performed to test for its potentially prognostic value. The Her2/neu (17q11.2) probe was included as a comparative diagnostic probe. This six-probe set was used to screen a cohort of 151 patients with Barrett's esophagus at the AMC using the automated CytoVision SPOT AX scoring system. Loss of p53 (17p13.1) occurred in 5% ofND, 9% of IND/LGD, and 46% of HGD (p<0.005, Fisher's exact test). Chromosome 17 gains were seen in 6% of ND, 21% of LGD, and 62% of HGD (p<0.05). Loss of p16 (9p21) was seen in 30% of ND, 20% of IND/LGD, and 46% of HGD/EAC (p<0.05). Ten percent of ND had loss of the Y chromosome, whereas 27% of HGD had loss of the Y chromosome (p<0.05). Amplification of Her2/neu (17q11.2) was detected in 62% of HGD (p<0.001). Based on these results, it was concluded that loss of p16 (9p21) and p53 (17p13.1) and polysomy of chromosomes 17 and 7 correlate with increasing grade of dysplasia in patients with Barrett's esophagus, and that these abnormalities are potentially prognostic for the progression of Barrett's esophagus (Rygiel et al. (2007), *supra*).

A total of 263 brush cytology specimens obtained from the AMC Barrett cohort were screened with the four probe set. The cohort included 197 brushings from ND cases, 19 from IND cases, 21 from LGD cases and 19 from HGD cases. The results were as shown in Table I.

**Table I**

| FISH abnormality | No./Total No. (%) | | | |
|---|---|---|---|---|
| | ND | IND | LGD | HGD |
| p16 (9p21) loss | 74/197 (37) | 5/19 (26) | 9/21 (42) | 7/19 (36) |
| p53 (17p13.1) loss | 7/197 (3.5) | 0 | 2/21 (9) | 6/19 (31) |
| CEP 7 loss | 2/197 (1) | 0 | 0 | 1/19 (5.2) |
| CEP 17 loss | 3/197 (1.5) | 1/19 (5) | 0 | 0 |
| CEP 7 tri- or tetrasomy | 5/197 (2.5) | 0 | 1/21 (9) | 2/19 (15) |
| CEP 17 tri- or tetrasomy | 9/197 (5) | 5/19 (26) | 4/21 (19) | 11/19 (57) |
| Her2/neu (17q11.2) amplification | 0 | 0 | 0 | 12/19 (63) |

In view of the foregoing, a prospective, long-term, follow-up of the value of the above markers for prognosticating HGD and/or EAC was conducted. The follow-up study is described herein in Examples 1 and 2.

The following terms are relevant to the present disclosure:
"About" refers to approximately a +/-10% variation from the stated value. It is to be understood that such a variation is always included in any given value provided herein, whether or not specific reference is made to it.
"Barrett's esophagus" is a condition of the esophagus in which normal, stratified, squamous epithelium is replaced with metaplasatic, premalignant, columnar type of epithelia, including the incompletely differentiated intestinal type, the cardiac type and the cardiac/oxyntic type in the distal esophagus.
"Biomarker," as defined by the National Institutes of Health, is "a characteristic that is objectively measured and evaluated as an indicator of normal biologic processes, pathogenic processes, or pharmacologic responses to a therapeutic intervention."
"Brush cytology specimen" is a sample of cells obtained by brushing an epithelial surface, e.g., gastrointestinal tract.
"Chromosome enumeration probe (CEP)" is any probe that enables the number of specific chromosomes in a cell to be enumerated. A chromosome enumeration probe typically recognizes and binds to a region near to (referred to as "peri-centromeric") or at the centromere of a specific chromosome, typically a repetitive DNA sequence (e.g., alpha satellite DNA). The centromere of a chromosome is typically considered to represent that chromosome, since the centromere is required for faithful segregation during cell division. Deletion or amplification of a particular chromosomal region can be differentiated from loss or gain of the whole chromosome (aneusomy), within which it normally resides, by comparing the number of signals corresponding to the particular locus (copy number) to the number of signals corresponding to the centromere. One method for making this comparison is to divide the number of signals representing the locus by the number of signals representing the centromere. Ratios of less than one indicate relative loss or deletion of the locus, and ratios greater than one indicate relative gain or amplification of the locus. Similarly, comparison can be made between two different loci on the same chromosome, for example on two different arms of the chromosome, to indicate imbalanced gains or losses within the chromosome. In lieu of a centromeric probe for a chromosome, one of skill in the art will recognize that a chromosomal arm probe may alternately be used to approximate whole chromosomal loss or gain. However, such probes are not as accurate at enumerating chromosomes, since the loss of signals for such probes may not always indicate a loss of the entire chromosome. Examples of chromosome enumeration probes include CEP® probes commercially available from Abbott Molecular, Inc., Des Plaines, IL (formerly Vysis, Inc., Downers Grove, IL).
"Copy number" is a measurement of DNA, whether of a single locus, one or more loci, or an entire genome. A "copy number" of two is "wild-type" in a human (because of diploidy, except for sex chromosomes). A "copy number" of other than two in a human (except for sex chromosomes) deviates from wild-type. Such deviations include amplifications, i.e., increases in copy numbers, and deletions, i.e., decreases in copy numbers and even the absence of copy numbers.
"Esophageal adenocarcinoma" is a malignancy of the esophagus arising from columnar type of cells that are present at the junction of the esophagus and the stomach, or in case of Barrett's esophagus in the distal esophagus.
"High-grade dysplasia" or "HGD" (also; High Grade Intraepithelial Neoplasia, 'HGIN') is a histological classification of a stage of more advanced expansion of immature epithelial cells, in this context Barrett's esophagus, with severe atypia of nuclei and cells and disturbed tissue architecture, mostly with a corresponding decrease in the number and location of mature cells. HGD often is indicative of the presence of an early neoplastic process or soon (<1 year) may lead to a neoplastic process, ie., esophageal adenocarcinoma.
"Indefinite for dysplasia" or "IND" is a condition that is in which there is uncertainty as to whether changes observed in the tissue are due to dysplasia or due to confounding factors such as inflammation and esophagitis caused by bile and acid reflux.
"Labeled," "labeled with a detectable label," and "detectably labeled" are used interchangeably herein to indicate that an entity (e.g., a probe) can be detected. "Label" and "detectable label" mean a moiety attached to an entity to render the entity detectable, such as a moiety attached to a probe to render the probe detectable upon binding to a target sequence. The moiety, itself, may not be detectable but may become detectable upon reaction with yet another moiety. Use of the term "detectably labeled" is intended to encompass such labeling. The detectable label can be selected such that the label generates a signal, which can be measured and the intensity of which is proportional to the amount of bound entity. A wide variety of systems for labeling and/or detecting molecules, such as nucleic acids, e.g., probes, are well-known. Labeled nucleic acids can be prepared by incorporating or conjugating a label that is directly or indirectly detectable by spectroscopic, photochemical, biochemical, immunochemical, electrical, optical, chemical or other means. Suitable detectable labels include radioisotopes, fluorophores, chromophores, chemiluminescent agents, microparticles, enzymes, magnetic particles, electron dense particles, mass labels, spin labels, haptens, and the like. Fluorophores and chemiluminescent agents are preferred herein.
"Locus-specific probe" refers to a probe that selectively binds to a specific locus in a region on a chromosome, e.g., a locus that has been determined to undergo gain/loss in metastasis. A probe can target coding or non-coding regions, or both, including exons, introns, and/or regulatory sequences, such as promoter sequences and the like.
"Low-grade dysplasia" or "LGD" (also: LGIN, Low Grade Intraepithelial Neoplasia) is an expansion of immature cells with a corresponding decrease in the number and location of mature cells and atypia of cell that can lead to development of HGD or to transform into esophageal adenocarcinoma. LDG is a preneoplastic stage that has a risk to progress to adenocarcinoma, which lies between that of ND and HGD.
"Negative for dysplasia" or "ND" means that there are no atypical/dysplastic changes in the cells, i.e., in the context of the present disclosure no atypical changes in the region of Barrett's esophagus.
"Nucleic acid sample" refers to a sample comprising nucleic acid in a form suitable for hybridization with a probe, such as a sample comprising nuclei or nucleic acids isolated or purified from such nuclei. The nucleic acid sample may comprise total or partial (e.g., particular chromosome(s)) genomic DNA, total or partial mRNA (e.g., particular chromosome(s) or gene(s)), or selected sequence(s). Condensed chromosomes (such as are present in interphase or metaphase) are suitable for use as targets in *in situ* hybridization, such as FISH.
"Predetermined cutoff' and "predetermined level" refer generally to a cutoff value that is used to assess diagnostic/prognostic/therapeutic efficacy results by comparing the assay results against the predetermined cutoff/level, where the predetermined cutoff/level already has been linked or associated with various clinical parameters (e.g., severity of disease, progression/nonprogression/improvement, etc.).
"Probe," in the context of the present disclosure, is an oligonucleotide or polynucleotide that can selectively hybridize to at least a portion of a target sequence under conditions that allow for or promote selective hybridization. In general, a probe can be complementary to the coding or sense (+) strand of DNA or complementary to the non-coding or anti-sense (-) strand of DNA (sometimes referred to as "reverse-complementary"). Probes can vary significantly in length. A length of about 10 to about 100 nucleotides, such as about 15 to about 75 nucleotides, e.g., about 15 to about 50 nucleotides, can be preferred.
"Prognosis of progression of Barrett's esophagus" is used herein to refer to the prognosis of the progression of Barrett's esophagus from one stage to another stage, such as from no dysplasia to low-grade dysplasia, low-grade dysplasia to high-grade dysplasia, or high-grade dysplasia to esophageal adenocarcinoma, and the like.
"Selectively hybridize to" (as well as "selective hybridization," specifically hybridize to," and "specific hybridization"), in the context of the present disclosure, refers to the binding, duplexing, or hybridizing of a nucleic acid molecule preferentially to a particular nucleotide sequence under stringent conditions. The term "stringent conditions" refers to conditions under which a probe will hybridize preferentially to its target sequence, and to a lesser extent to, or not at all to, other non-target sequences. A "stringent hybridization" and "stringent hybridization wash conditions" in the context of nucleic acid hybridization (e.g., as in array, Southern hybridization, Northern hybridization, or FISH) are sequence-dependent, and differ under different conditions. An extensive guide to the hybridization of nucleic acids is found in, e.g., Tijssen, Laboratory Techniques in Biochemistry and Molecular Biology--Hybridization with Nucleic Acid Probes, Part I, Ch. 2, "Overview of principles of hybridization and the strategy of nucleic acid probe assays," Elsevier, NY (1993) ("Tijssen"). Generally, highly stringent hybridization and wash conditions are selected to be about 5 °C lower than the thermal melting point (Tₘ) for the specific sequence at a defined ionic strength and pH. The Tₘ is the temperature (under defined ionic strength and pH) at which 50% of the target sequence hybridizes to a perfectly matched probe. Very stringent conditions are selected to be equal to the Tₘ for a particular probe. An example of stringent hybridization conditions for hybridization of complementary nucleic acids, which have more than 100 complementary residues, on an array or on a filter in a Southern or Northern blot is 42 °C using standard hybridization solutions (see, e.g., Sambrook and Russell, Molecular Cloning: A Laboratory Manual, 3rd ed., Vol. 1-3, Cold Spring Harbor Laboratory, Cold Spring Harbor Press, NY (2001)).
"Target sequence," "target region," and "nucleic acid target" refer to a nucleotide sequence that resides at a specific chromosomal location whose loss and/or gain, for example, is being determined.

The terminology used herein is for the purpose of describing particular embodiments only and is not otherwise intended to be limiting.

The detection of biomarkers can be used as indicators for progression from Barrett's esophagus to esophageal adenocarcinoma, i.e., as prognostic biomarkers, and as indicators of the stage of early or advanced neoplasia, i.e., as diagnostic biomarkers. The progression from Barrett's esophagus to esophageal adenocarcinoma is associated with the occurrence of several genetic changes.

### Methods ofprognosticating the progression of Barrett's esophagus

A method of prognosticating progression of Barrett's esophagus in a patient is provided. The method comprises detecting in a sample of esophageal cells from the patient at least one abnormality selected from the group consisting of p16 loss, p53 loss, and chromosome 7 aneuploidy and chromosome 17 aneuploidy. The presence of at least p16 or p53 loss or aneuploidy for both 7 and 17 indicates that Barrett's esophagus will likely progress towards LGD, HGD or esophageal adenocarcinoma. Preferably, at least one abnormality is p16 loss. More preferably, at least one abnormality is p16 loss, chromosome 7 aneuploidy, and chromosome 17 aneuploidy. The method can further comprise detecting in the sample of esophageal cells from the patient at least one abnormality selected from the group consisting of 20q gain, C-myc gain, and Her2 gain. In this regard, 20q gain, C-myc gain, and Her2 gain can be diagnostic of HGD.

The sample of esophageal cells can be obtained by any suitable method known in the art. Preferably, the sample of esophageal cells is obtained by a brush cytology method. Use of brush cytology specimens enables the use of an automated system, such as the CytoVision SPOT AX, for assessment of abnormalities.

The above method can be carried out using any suitable detection method known in the art. Preferably, the above method is carried out using *in situ* hybridization, such as fluorescent *in situ* hybridization (FISH). Preferably, each probe is detectably labeled with a distinct label, such as a distinct fluorophore.

When the above methods are carried out by *in situ* hybridization, in which each probe is detectably labeled with a distinct label, such as by FISH, in which each probe is labeled with a distinct fluorophore, the methods are typically carried out on a sample of esophageal cells, which are fresh (fresh cells can be cultured for 1-3 days and a blocker, such as Colcemid, can be added to the culture to block the cells in metaphase, during which chromosomes are highly condensed and can be visualized), frozen, or fixed (e.g., fixed in cytopreserve, formalin and embedded in paraffin), treated (e.g., with RNase and pepsin) to increase accessibility of target nucleic acid (e.g., DNA) and reduce non-specific binding, and then subjected to hybridization with one or more probes, washing to remove any unbound probes, and detection of hybridized probes. For example, a cell suspension can be applied as a single layer onto a slide, and the cell density can be measured by a light or phase contrast microscope. A section (approximately 5 µm in thickness) of a formalin-fixed, paraffin-embedded sample of esophageal cells can be mounted onto a slide, such as a SuperFrost Plus positively charged slide (available from ThermoShandon, Pittsburgh, PA), baked at 56 °C overnight, de-paraffinized, submerged in 1 x saline sodium citrate, pH 6.3, at 80 °C for 35 minutes, and washed in water for three minutes. After protease digestion (4 mg pepsin/mL and 0.2 N HCl) at 37 °C for 15 minutes, the section can be rinsed in water for three minutes, passed through graded ethanol, and dried. Preferably, hybridization with one or more probes as described above is carried out at 37 °C for 16-18 hours in an automated co-denaturation oven (HYBrite or ThermoBrite Denaturation/Hybridization System, Abbot Molecular, Inc., Des Plaines, IL) according to the manufacturer's instructions (such methods typically involve denaturation of probes and target nucleic acids). After hybridization, the section is preferably placed in washing buffer (2 x saline sodium citrate/0.3% NP40; available from Abbott Molecular, Inc.) at room temperature for 2-10 minutes to remove the coverslip and then immersed in 73 °C washing buffer for two minutes, dried, and mounted with 4'6'-diamidino-2-phenylindole dihydrochloride hydrate (DAPI) I anti-fade solution (Abbott Molecular, Inc.). Preferably, the slide is analyzed with an epi-fluorescence microscope equipped with single band-pass filters (Abbott Molecular, Inc.).

Prior to detection, cell samples may be optionally pre-selected based on apparent cytologic abnormalities. Pre-selection identifies suspicious cells, thereby allowing the screening to be focused on those cells. In another embodiment, pre-selection may provide for the exclusion of squamous cells from specimens. During pre-selection, cells from a biological sample can be placed on a microscope slide and visually scanned for cytologic abnormalities commonly associated with dysplastic and neoplastic cells. Such abnormalities include abnormalities in nuclear size, nuclear shape, and nuclear staining, as assessed by counterstaining nuclei with nucleic acid stains or dyes such as propidium iodide or 4,6-diamidino-2-phenylindole dihydrochloride (DAPI) usually following hybridization of probes to their target DNAs. Typically, neoplastic cells harbor nuclei that are enlarged, irregular in shape, and/or show a mottled staining pattern. Propidium iodide, typically used at a concentration of about 0.4 µg/ml to about 5 µg/ml, is a red-fluorescing DNA-specific dye that can be observed at an emission peak wavelength of 614 nm. DAPI, typically used at a concentration of about 125 ng/ml to about 1,000 ng/ml, is a blue fluorescing DNA-specific stain that can be observed at an emission peak wavelength of 452 nm with a DAPI filter at low magnification. In this case, only those cells pre-selected for detection are subjected to counting for chromosomal losses and/or gains. In one embodiment, pre-selected cells on the order of at least 20, or in another embodiment, at least 30-40, in number are chosen for assessing chromosomal losses and/or gains.

Alternatively, an area evidencing some level of dysplasia or a suspicious lesion can be localized using the DAPI filter at low magnification and thoroughly inspected for the presence of nuclei harboring abnormal copy numbers of any probe. In a normal cell, two copies of a given probe will be detected. In an abnormal cell, more or less copies of a given probe will be detected. Areas with the most significant copy number changes are preferably selected for enumeration. Wherever possible, three abnormal areas are selected and, within each abnormal area, 10 random nuclei are analyzed under high power (64 x or 100 x objective). Preferably, nuclei are non-overlapping and harbor sufficiently bright signals.

Alternatively, cells for detection may be chosen independent of cytologic or histologic features. For example, all non-overlapping cells in a given area or areas on a microscope slide may be assessed for chromosomal losses and/or gains. As a further example, cells on the slide, e.g., cells that show altered morphology, on the order of at least about 50, and more preferably at least about 100, in number that appear in consecutive order on a microscope slide may be chosen for assessing chromosomal losses and/or gains.

The copies of at least one of p16, p53, chromosome 7, and chromosome 17 are counted. Preferably, at least the copy number of p16 is counted. More preferably, the copy numbers of p16, chromosome 7, and chromosome 17 are counted. In addition the copies of at least one of 20q, C-myc, and Her2 is/are counted. The copy numbers can be compared to pre-determined cut-off(s). p16 loss, p53 loss, chromosome 7 aneuploidy, and chromosome 17 aneuploidy indicate that Barrett's esophagus will likely progress towards esophageal adenocarcinoma. Additionally, 20q gain, C-myc gain, and Her2 gain indicate that Barrett's esophagus will likely progress towards esophageal adenocarcinoma.

Thus, such methods comprise contacting a sample of Barrett's esophagus from a patient, e.g., a nucleic acid sample, with at least one probe that binds selectively to a target nucleic acid sequence (i.e., at least one of p16, p53, chromosome 7, and chromosome 17, such as at least p16, alone or in further combination with chromosome 7 and chromosome 17, wherein such a combination can further include at least one of 20q, C-myc, and Her2) under conditions that allow (or promote) the probe to bind selectively with its target nucleic acid sequence and form a stable hybridization complex. Such methods further comprise detecting the formation of the hybridization complex and counting the number of hybridization complexes. In view of the number of hybridization complexes comprising p16, p53, chromosome 7, chromosome 17, 20q, C-myc, and/or Her2, the method further comprises determining the copy number of p16, p53, chromosome 7, chromosome 17, 20q, C-myc, and/or Her2. The copy number can be compared to a pre-determined cut-off, wherein a copy number greater than the pre-determined cut-off (i.e., for a gain) and a copy number less than the pre-determined cut-off (i.e., for a loss), as appropriate, indicates that progression of Barrett's esophagus will likely occur. In one embodiment, the cut-off is determined on counts of copy number found in normal squamous epithelial cells.

Other methods already known in the art or currently under development can be used. Such methods may necessitate the use of a sample of Barrett's esophagus that is other than a section of a Barrett's esophagus that is fixed in formalin and embedded in paraffin, e.g., a fresh or frozen section of Barrett's esophagus, homogenized cells from Barrett's esophagus, lysed cells from Barrett's esophagus, or isolated or purified nucleic acids (e.g., a "nucleic acid sample" such as DNA) from Barrett's esophagus ("sample of Barrett's esophagus" as used herein is intended to encompass all forms of a sample of Barrett's esophagus that enable the determination of copy number and gain/loss). In this regard, a touch preparation (a monolayer of cells obtained by pressing fresh or frozen tissue against a slide) prepared from an uncultured sample of Barrett's esophagus can be used (see, e.g., Kallioniemi et al., Cytogenet. Cell Genet. 60: 190-193 (1992)). Touch preparations contain intact nucleic and do not suffer from the truncation artifact of sectioning. The monolayer of cells in a touch preparation may be fixed, e.g., in alcohol, such as ethanol, or alcoholic solution, such as 3:1 methanol:acetic acid. Nuclei also can be extracted from thick sections of paraffin-embedded specimens to reduce truncation artifacts and eliminate extraneous embedded material. Typically, biological samples, once obtained, are harvested and processed prior to hybridization using standard methods known in the art. Such processing typically includes protease treatment and additional fixation in an aldehyde solution, such as formaldehyde.

Examples of methods that can be used herein include, but are not limited to, quantitative polymerase chain reaction (Q-PCR), real-time Q-PCR (Applied Biosystems, Foster City, CA), densitometric scanning of PCR products, digital PCR, optionally with pre-amplification of the gene(s) and/or chromosomal region(s) for which copy number(s) is/are to be determined (see, e.g., Vogelstein et al., PNAS USA 96: 9236-9241 (1999); U.S. Pat. App. Pub. No. 2005/0252773; and U.S. Pat. App. Pub. No. 2009/0069194), comparative genomic hybridization (CGH; see, e.g., Kallioniemi et al., Science 258: 818-821 (1992); and Int'1 Pat. App. Pub. No. WO 93/18186), microsatellite or Southern allelotype analysis, dot blots, arrays, microarrays (Carter, Nature Genetics Supplement 39: S16-S21 (July 2007)), multiplex amplifiable probe hybridization (MAPH), multiplex ligation-dependent probe amplification (MLPA; see, e.g., Schouten et al., Nucleic Acids Res. 30: e 57 (2002)), denaturing high performance liquid chromatography (dHPLC; Kumar et al., J. Biochem. Biophys. Methods 64(3): 226-234 (2005)), dynamic allele-specific hybridization (DASH), measuring fluorescent probe lengths on combed genomic DNA (Herrick et al., PNAS 97(1): 222-227 (2000)), reference query pyrosequencing (RQPS; Liu et al., Cold Spring Harb. Protoc. doi: 10.1101/pdb.prot5491 (2010)), mapping of fosmid ends onto a reference sequence (capillary-based technology), microelectrophoretic and nanopore sequencing (see, e.g., Service, Science 311: 1544-1546 (2006); and Shendure et al., Nat. Rev. Genet. 5: 335-344 (2004)), and the like.

Denaturation of nucleic acid targets for analysis by *in situ* hybridization and similar methods typically is done in such a manner as to preserve cell morphology. For example, chromosomal DNA can be denatured by high pH, heat (e.g., temperatures from about 70-95 °C), organic solvents (e.g., formamide), and combinations thereof. Probes, on the other hand, can be denatured by heat in a matter of minutes.

After denaturation, hybridization is carried out. Conditions for specifically hybridizing the probes to their nucleic acid targets generally include the combinations of conditions that are employable in a given hybridization procedure to produce specific hybrids, the conditions of which may easily be determined by one of ordinary skill in the art. Such conditions typically involve controlled temperature, liquid phase, and contact between a probe and a target. Hybridization conditions vary depending upon many factors including probe concentration, target length, target and probe G-C content, solvent composition, temperature, and duration of incubation. At least one denaturation step may precede contact of the probes with the targets. Alternatively, the probe and the target may be subjected to denaturing conditions together while in contact with one another, or with subsequent contact of the probe with the biological sample. Hybridization may be achieved with subsequent incubation of the probe/sample in, for example, a liquid phase of about a 50:50 volume ratio mixture of 2-4 x SSC and formamide, at a temperature in the range of about 25 to about 55° C for a time that is illustratively in the range of about 0.5 to about 96 hours, or more preferably at a temperature of about 32 to about 40° C for a time in the range of about 2 to about 16 hours. In order to increase specificity, use of a blocking agent, such as unlabeled blocking nucleic acid, as described in U.S. Pat. No. 5,756,696 (the contents of which are herein incorporated by reference in their entirety, and specifically for the description of the use of blocking nucleic acid), may be used. Other conditions may be readily employed for specifically hybridizing the probes to their nucleic acid targets present in the sample, as would be readily apparent to one of skill in the art. Hybridization protocols are described, for example, in Pinket et al., PNAS USA 85: 9138-9142 (1988); In situ Hybridization Protocols, Methods in Molecular Biology, Vol. 33, Choo, ed., Humana Press, Totowa, NJ (1994); and Kallioniemi et al., PNAS USA 89: 5321-5325 (1992).

Upon completion of a suitable incubation period, non-specific binding of chromosomal probes to sample DNA may be removed by a series of washes. Temperature and salt concentrations are suitably chosen for a desired stringency. The level of stringency required depends on the complexity of a specific probe sequence in relation to the genomic sequence, and may be determined by systematically hybridizing probes to samples of known genetic composition. In general, high stringency washes may be carried out at a temperature in the range of about 65 to about 80° C with about 0.2 x to about 2 x SSC and about 0.1% to about 1% of a non-ionic detergent, such as Nonidet P-40 (NP40). If lower stringency washes are required, the washes may be carried out at a lower temperature with an increased concentration of salt.

When fluorophore-labeled probes or probe compositions are used, the detection method can involve fluorescence microscopy, flow cytometry, or other means for determining probe hybridization. Any suitable microscopic imaging method may be used in conjunction with the methods described herein for observing multiple fluorophores. In the case where fluorescence microscopy is employed, hybridized samples may be viewed under light suitable for excitation of each fluorophore and with the use of an appropriate filter or filters. Automated digital imaging systems, such as the MetaSystems, BioView or Applied Imaging systems, may alternatively be used.

Depending on the method employed, a digital image analysis system can be used to facilitate the display of results and to improve the sensitivity of detecting small differences in fluorescence intensity. An exemplary system is QUIPS (an acronym for quantitative image processing system), which is an automated image analysis system based on a standard fluorescence microscope equipped with an automated stage, focus control and filter wheel (Ludl Electronic Products, Ltd., Hawthorne, NY). The filter wheel is mounted in the fluorescence excitation path of the microscope for selection of the excitation wavelength. Special filters (Chroma Technology, Brattleboro, VT) in the dichroic block allow excitation of the multiple dyes without image registration shift. The microscope has two camera ports, one of which has an intensified CCD camera (Quantex Corp., Sunnyvale, Calif.) for sensitive high-speed video image display, which is used for finding interesting areas on a slide as well as for focusing. The other camera port has a cooled CCD camera (model 200 by Photometrics Ltd., Tucson, AZ), which is used for the actual image acquisition at high resolution and sensitivity. The cooled CCD camera is interfaced to a SUN 4/330 workstation (SUN Microsystems, Inc., Mountain View, CA) through a VME bus. The entire acquisition of multicolor images is controlled using an image processing software package SCIL-Image (Delft Centre for Image Processing, Delft, Netherlands).

In array CGH (aCGH) the probes are immobilized at distinct locations on a substrate and are not labeled (see, e.g., Int'l Pat. App. Pub. No. WO 96/17958). Instead, sample nucleic acids, which comprise target nucleic acid(s), are labeled. Either the sample nucleic acids are labeled prior to hybridization or the hybridization complexes are detectably labeled. In dual- or multi-color aCGH the probe array is simultaneously or sequentially hybridized to two or more collections of differently labeled target nucleic acids.

### Probes

In view of the above, also provided is a set of probes that enables prognosis of progression of Barrett's esophagus in a patient. The set of probes comprises a probe for p16 (loss), a probe for p53 (loss), a probe for chromosome 7 (aneuploidy), and a probe for chromosome 17 (aneuploidy). The set of probes can comprise at least one additional probe. The at least one additional probe can be selected from the group consisting of a probe for 20q (gain), a probe for C-myc (gain), and a probe for Her2 (gain). Thus, the set of probes can comprise a probe for p16 (loss), a probe for p53 (loss), a probe for chromosome 7 (aneuploidy), a probe for chromosome 17 (aneuploidy), a probe for 20q (gain), a probe for c-myc (gain), and a probe for Her2 (gain).

The probe for chromosome 7 can hybridize to the alpha satellite DNA located at the centromere of chromosome 7, whereas the probe for chromosome 17 can hybridize to the alpha satellite DNA located at the centromere of chromosome 17. Examples of such probes include CEP7 and CEP17. The probe for p16 can hybridize to the locus specific site 9p21, the probe for p53 can be to the 17p13.1 region, the probe for 20q can be to 20q13, the probe for C-myc can be Vysis LIS C-MYC to 8q24, and the probe for Her2 can be to 17q11-q12 chromosomal region. In one embodiment, the probe for p16 hybridizes to all or a portion of the p16 gene at 9p21 on chromosome 9. In another embodiment, the probe hybridizes to the the entire p16 gene on chromosome 9 and adjacent regions, extending from a point centromeric of the p16 gene to a point telomeric of the p16 gene.

In one embodiment, the probe for p53 hybridizes to all or a portion of the the p53 gene at 17p13.1 on chromosome 17. In another embodiment, the probe hybridizes to the the entire p53 gene on chromosome 17 and adjacent regions, extending from a point centromeric of the p53 gene to a point telomeric of the p53 gene.

The probe for 20q can hybridize on any appropriate location of the q arm of chromosome 20. In one embodiment, the probe for 20q hybridizes to all or a portion of the AURKA gene on chromosome 20. In another embodiment, the probe for 20q hybridizes to the the entire AURKA gene on chromosome 20 and adjacent regions, extending from a point centromeric of the AURKA gene to a point telomeric of the AURKA gene. In another embodiment, the probe for 20q hybridizes to the to the 20q13.2 region of chromosome 20 and includes the 17.5 kb ZNF217 gene. In another embodiment, the probe for 20q hybridizes to all or a portion of the D20S108 locus located on chromosome 20q12. In another embodiment, the probe for 20q hybridizes to the entire D20S108 locus on chromosome 20 and adjacent regions, extending from a point centromeric of the D20S108 locus to a point telomeric of the D20S108 locus.

In one embodiment, the probe for C-myc hybridizes to all or a portion of the c-myc gene located in the 8q24.12-q24.13 region of chromosome 8.

In one embodiment, the probe for Her2 hybridizes to all or a portion of the her2/neu / erbB2 gene at 17q11.2-12 region of chromosome 17. In another embodiment, the probe for Her2 hybridizes to the entire her2/neu / erbB2 gene and adjacent regions, extending from a point centromeric of the her2/neu / erbB2 gene to a point telomeric of the her2/neu / erbB2 gene.

Chromosome enumerator probes (CEP) and locus-specific probes that target a chromosome region or subregion can be obtained commercially or readily prepared by those in the art. Such probes can be commercially obtained from Abbott Molecular, Inc. (Des Plaines, IL), Molecular Probes, Inc. (Eugene, OR), or Cytocell (Oxfordshire, UK). Chromosomal probes can be prepared, for example, from protein nucleic acids (PNA), cloned human DNA such as plasmids, bacterial artificial chromosomes (BACs), and PI artificial chromosomes (PACs) that contain inserts of human DNA sequences. A region of interest can be obtained via PCR amplification or cloning. Alternatively, chromosomal probes can be prepared synthetically in accordance with methods known in the art. In another embodiment, the chromosomal probes can be oligo probes.

When targeting of a particular gene locus is desired, probes that hybridize along the entire length of the targeted gene can be preferred, although not required. A locus-specific probe can be designed to hybridize to an oncogene or tumor suppressor gene, the genetic aberration of which is correlated with metastasis, e.g., p16, p53, C-myc and Her2.

Preferably, probes are detectably labeled, and each probe is distinctly labeled. Preferably, the probes are detectably labeled with fluorophores, and each probe is distinctly labeled. Examples of preferred fluorophores include, but are not limited to, 7-amino-4-methylcoumarin-3-acetic acid (AMCA), 5-carboxy-X-rhodamine, 6-carboxy-X-rhodamine, lissamine rhodamine B, 5-carboxyfluorescein, 6-carboxyfluorescein, fluorescein-5-isothiocyanate (FITC), 7-diethylaminocoumarin-3-carboxylic acid, tetramethylrhodamine-5-isothiocyanate, tetramethylrhodamine-6-isothiocyanate, 5-carboxyltetramethylrhodamine, 6-carboxytetramethylrhodamine, 7-hydroxycoumarin-3-carboxylic acid, N-4,4-difluoro-5,7-dimethy-4-bora-3a,4a-diaza-3-indacenepropionic acid, eosin-5-isothiocyanate, erythrosine-5-isothiocyanate, SpectrumRed (Abbott Molecular, Inc.), SpectrumGold (Abbott Molecular, Inc.), SpectrumGreen (Abbott Molecular, Inc.), SpectrumAqua (Abbott Molecular, Inc.), TEXAS RED (Molecular Probes, Inc.), and CASCADE blue acetylazide (Molecular Probes, Inc.). The particular label used is not critical; desirably, however, the particular label does not interfere with *in situ* hybridization of the probe. The label desirably is detectable in as low copy number as possible to maximize the sensitivity of the assay and be detectable above any background signal. Also desirably, the label provides a highly localized signal, thereby providing a high degree of spatial resolution.

Attachment of fluorophores to nucleic acid probes is well-known in the art and can be accomplished by any available means. Fluorophores can be covalently attached to a particular nucleotide, for example, and the labeled nucleotide incorporated into the probe using standard techniques such as nick translation, random priming (Rigby et al., J. Mol. Biol. 113: 237 (1997)), PCR labeling, direct labeling by chemical modification of particular residues, such as cytosine residues (U.S. Pat. No. 5,491,224), and the like. Alternatively, the fluorophore can be covalently attached via a linker to the deoxycytidine nucleotides of the probe that have been transaminated. Methods for labeling probes are described in U.S. Pat. No. 5,491,224, and Morrison et al., Molecular Cytogenetics: Protocols and Applications, Chapter 2, "Labeling Fluorescence In Situ Hybridization Probes for Genomic Targets," pp. 21-40, Fan, Ed., Humana Press (2002), both of which are herein incorporated by reference for their descriptions of labeling probes.

One of skill in the art will recognize that other agents or dyes can be used in lieu of fluorophores as label-containing moieties. Luminescent agents include, for example, radioluminescent, chemiluminescent, bioluminescent, and phosphorescent label-containing moieties. Alternatively, detection moieties that are visualized by indirect means can be used. For example, probes can be labeled with biotin or digoxygenin using routine methods known in the art, and then further processed for detection. Visualization of a biotin-containing probe can be achieved via subsequent binding of avidin conjugated to a detectable marker. The detectable marker may be a fluorophore, in which case visualization and discrimination of probes can be achieved as described below.

Chromosomal probes hybridized to target regions may alternatively be visualized by enzymatic reactions of label moieties with suitable substrates for the production of insoluble color products. Each probe may be discriminated from other probes within the set by choice of a distinct label moiety. A biotin-containing probe within a set may be detected via subsequent incubation with avidin conjugated to alkaline phosphatase (AP) or horseradish peroxidase (HRP) and a suitable substrate. 5-bromo-4-chloro-3-indolylphosphate and nitro blue tetrazolium (NBT) serve as substrates for alkaline phosphatase, while diaminobenzoate serves as a substrate for HRP.

### Kits

Also provided is a kit. The kit comprises (a) a set of probes that enables prognosis of progression of Barrett's esophagus in a patient and instructions for prognosticating the progression of Barrett's esophagus in a patient. The set of probes comprises a probe for p16, a probe for p53, a probe for chromosome 7, and a probe for chromosome 17. The instructions comprise determining in a sample of esophageal cells from a patient at least one abnormality selected from the group consisting of p16 loss, p53 loss, chromosome 7 aneuploidy, and chromosome 17 aneuploidy. The set of probes can comprise at least one additional probe, and the instructions can comprise further instructions. The at least one additional probe can be selected from the group consisting of a probe for 20q, a probe for C-myc, and a probe for Her2. The further instructions can comprise determining in a sample of esophageal cells from a patient at least one additional abnormality selected from the group consisting of 20q gain, C-myc gain, and Her2 gain. Such kits may further comprise blocking agents or probes, various labels or labeling agents to facilitate detection of the probes, reagents for hybridization (e.g., buffers), a metaphase spread, and the like.

### EXAMPLES

The following examples serve to illustrate the present invention. The examples are not intended to limit the scope of the invention as later claimed. Examples not falling within the scope of the claims are given for reference only.

### Example 1

This example describes a prospective, long-term, follow-up of markers for prognosticating LGD, HGD and/or EAC in an academic cohort of Barrett's esophagus (BE) patients.

The clinical problem of BE is that the vast majority of patients have no dysplasia and a risk of <0.5% per year to progress. In this study the aim was to identify early prognostic markers that can be used to identify those patients with BE and no dysplasia and who are staged as indefinite for dysplasia in biopsy specimens. The AMC cohort, which had been screened for the FISH markers p16 (9p21) loss, p53 (17p13.1) loss, chromosome 7 loss/gain (using chromosome enumerator probe (CEP) 7), chromosome 17 loss/gain (using CEP 17), and Her2/neu (17q11.2) gains/amplification between 2002 and 2008, was prospectively followed for a median follow-up period of 60 months. Patients with LGD, HGD or EAC at the index endoscopy or histological progression within six month and/or previous treatment for BE were excluded. Patients, who had previous treatment by endoscopic mucosal resection (EMR) and/or an ablation therapy (e.g., radiofrequency ablation) for treatment of BE, were also excluded. At index endoscopy (t=0) biopsies were taken following ACG guidelines, and brush cytology was performed. All patients underwent prospective surveillance. Histology and FISH were performed on cytology samples independently and blinded. Histology was assessed during surveillance endoscopy performed by two dedicated endoscopists, who followed the current criteria and biopsy protocol (i.e., 4-quadrant biopsies of each centimeter of the Barrett segment and of any suspicious lesions). Histopathology was assessed by one expert academic gastrointestinal (GI) pathologist and, in the case of dysplasia, by two expert academic GI pathologists. The endpoints were histological progression of ND/IND into LGD/HGD/EAC, or a FU of 60 months. The assay tested as "marker-positive" when the percentage of abnormal cells with genetic abnormalities for Her2, p16 or p53 and/or aneuploidy (aneusomy for chromosomes 7 and 17) were higher than the cut-off values. Cut off values were determined by counting brush cytology specimens of normal squamous esophageal epithelium (mean +3SD).

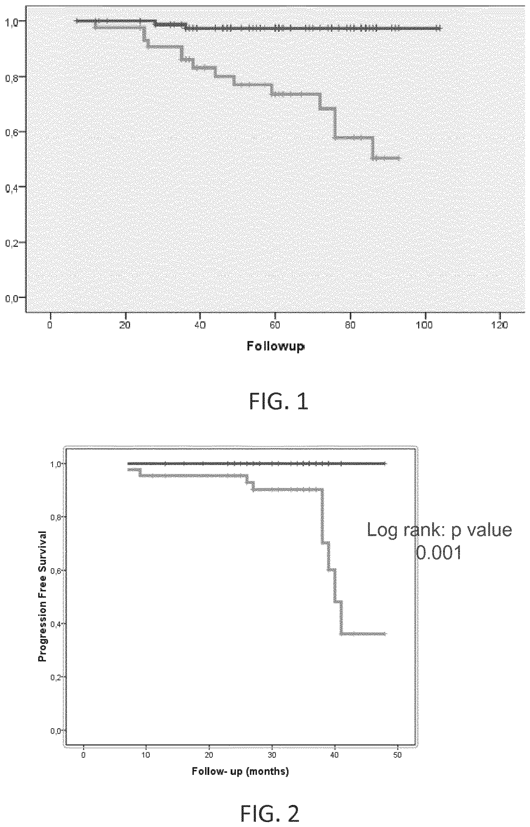

In view of the above, 195 patients were included in the study. One hundred seventy six (176) of the 195 patients reached an endpoint, and 140 of those were males (80%). Mean age at entry was 62(SD±12). Mean Barrett M- and C-lengths were 3.6 cm (SD±2.5) and 1.16 cm (SD±1), respectively. Seventeen patients progressed from ND/IND to LGD or HGD/EAC. The FISH assay tested positive in 55/176 (31%). Fifteen of the 17 patients (15/55 (27%)), who progressed from ND/IND to LGD or HGD/EAC, were marker-positive, while only two patients (2/121(1.6%)) who progressed from ND/IND to LGD or HGD/EAC, were marker-negative. Kaplan Meier curves showed that the progression-free interval was significantly shorter in the marker-positive group versus the marker-negative group (p=0.001; Figure 1). A multivariate analysis using a Cox regression model (Table 1) showed that a positive test correlated with an HR of 10.6 (p=0.003) to progress when compared to other variables.

In this prospective long term follow-up study it was demonstrated that the FISH biomarker assay of cytology specimens of BE surveillance patients, as described herein, correlates with a 10.6 increased risk for progression. This assay shows promise as a useful tool to improve the risk stratification of BE patients and increase the efficacy of surveillance.

As shown in Fig. 1, which is a graph of cumulative rate of progression free survival vs. follow-up (months) showing the use of the Kaplan Meier curve to represent the follow-up data for the progression of patient's with Barrett's esophagus, in which the top line represents the 'marker-negative' group and the bottom line represents the 'marker-positive' group, the marker-positive group showed a progression in the stage of dysplasia (see drops in the bottom line). The difference in progression of the two groups over time is statistically significant (log-rank test, p-value=0.01).

### Table II: Multivariate analysis using Cox regression model

Variables
Hazard Ratio
(Exp (B))
95,0% CI (Exp (B))
Significance

Lower
Upper

Markers
10,644
2,269
49,921
0,003

Age
1,012
0,966
1,06
0,616

Barrett C length
1,001
0,655
1,53
0,996

Barrett M length
1,001
0,655
1,53
0,996

Height
0,974
0,906
1,046
0,465

BMI
0,996
0,939
1,056
0,884

Smoking
1,38
0,403
4,726
0,608

Alcohol
1,278
0,137
11,879
0,829

### Example 2

This example describes the validation of the prognostic DNA FISH marker set in a random community-based BE surveillance population.

The study was initiated because the combination of CEP 7, 17, Her2/neu (17q11.2) gains/amplification, p53 (17p13.1), and p16 (9p21) losses promised to have prognostic value to identify those Barrett patients who have an increased risk to develop dysplasia in a tertiary referral center. Between January 2007 and November 2010 brush cytology specimens were prospectively and randomly collected at six regional hospitals in the Amsterdam/North Holland region from 266 BE patients, who were diagnosed with no dysplasia. Patients with LGD, HGD or EAC at the index endoscopy or histological progression within six months and/or previous treatment for BE were excluded. Patients, who had previous treatment by endoscopic mucosal resection (EMR) and/or an ablation therapy (e.g., radiofrequency ablation) for treatment of BE, were also excluded. At index endoscopy (t=0) biopsies were taken following ACG guidelines, and brush cytology was performed. All patients underwent prospective surveillance. Histology and FISH on cytology samples were performed independently and blinded. Biopsies were processed routinely for histopathological staging of dysplasia. Brush specimens were taken from the normal squamous epithelium and the Barrett segment. FISH with the marker set was carried out on the cytology specimens, and results were evaluated with respect to histology. The assay tested as "marker-positive" when the percentage of abnormal cells with genetic abnormalities for Her2 (gains/amplification), p16 or p53 and/or aneuploidy (aneusomy for chromosomes 7 and 17) were higher than the cut-off values. Cut-off values used for each probe to detect an abnormal FISH result in the Barrett cytology specimens were determined in the cytology samples of the normal squamous mucosa. Finally FISH results were correlated with histological stage.

Presently, 170 patients have reached an average follow up of 48 months. The mean age of the 170 patients is 64 (range of 32-88), and 81% (218/266) of the patients are male. The maximum Barrett segment length is 1.16 cm (range: 1-3cm). At t=0 the FISH assay tested positive in 48/170 (28%) cases. Ten of the 170 patients showed histological progression, and all were marker-positive. Eight patients progressed from ND to LGD, while two patients progressed from ND to HGD. Using Kaplan Meier curves to evaluate progression-free survival, a highly significant difference was observed between the two groups (log-rank test, p-value 0.001, Fig. 2). Cox regression analysis revealed a HR of 9.1 (p-value 0.001) to progress in case of a positive test as compared to other variables (Table III). The data indicate that the FISH marker assay has high prognostic value to predict progression in BE patients. The data support the use of the DNA FISH biomarker assay to increase the cost efficacy of Barrett surveillance patients.

**Table III: Multivariate Analysis using Cox regression Model**

| Variables | Hazard Ratio (Exp (B)) | 95 % CI | | Significance |
|---|---|---|---|---|
| | | lower | Upper | |
| Markers | 9.175 | 2.68 | 25.02 | 0.001 |
| Age | 0.937 | 0.86 | 1.02 | 0.136 |
| Barrett C Length | 1.017 | 0.65 | 2.56 | 0.770 |
| Barrett M Length | 1.172 | 0.40 | 3.40 | 0.770 |

### Example 3

This example describes how brush cytology of the entire segment of the Barrett mucosa, in combination with biomarkers, can reduce sampling errors and increase accuracy of the identification of HGD/early EAC.

Despite careful endoscopic examination of Barrett esophagus and biopsies taken during surveillance programs, lesions with HGD or early esophageal adenocarcinoma (EAC) can still be missed. Earlier studies have shown that aneuploidy and amplifications of the oncogenes C-Myc, Her-2 and 20q are frequent events in HGD and EAC. These markers can be determined efficiently by FISH on brush cytology specimens of BE patients.

Abnormalities of Her2/Neu, c-Myc and 20q13.2 and aneuploidy (determined by aneusomy for chromosomes 7 and 17) were analyzed for their accuracy for detecting HGD/EAC in an academic tertiary referral cohort of 202 BE and EAC patients. The marker set was applied on a community cohort of 305 BE patients from 6 different hospitals. At t=0, biopsies and brush cytology specimens were obtained from all patients. Biopsies were used for staging of dysplasia. FISH for the five-marker set was carried out on the cytology specimens. Histological and FISH evaluations were performed blindly, by independent investigators. Finally, the FISH results were evaluated for their accuracy to detect HGD/EAC.

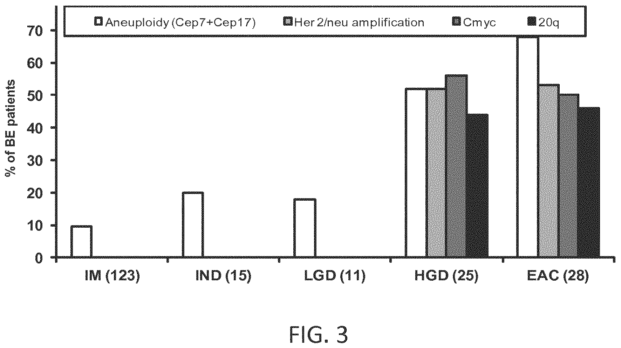

Of 202 cases 138 were ND/IND, 11 were LGD, 53 were HGD /EAC. Aneuploidy was found in 32/53 (60%) but also in increasing frequency in ND/IND to LGD (ND (10%), IND (20%), LGD (18%)). Amplifications of Her2/Neu (17ql 1.2), c-Myc (8q24) and 20q13.2 were not present in ND/IND or LGD cases. In the HGD/EAC cases amplification of Her-2/Neu was found in 28/53 (53%). Amplification of C-Myc was seen in 28/53 (53%)) and amplification of 20q was seen in 24/53 (45%) of the HGD/EAC cases (Fig. 3).

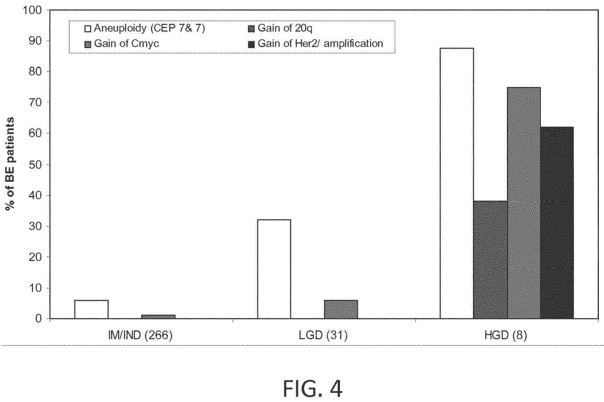

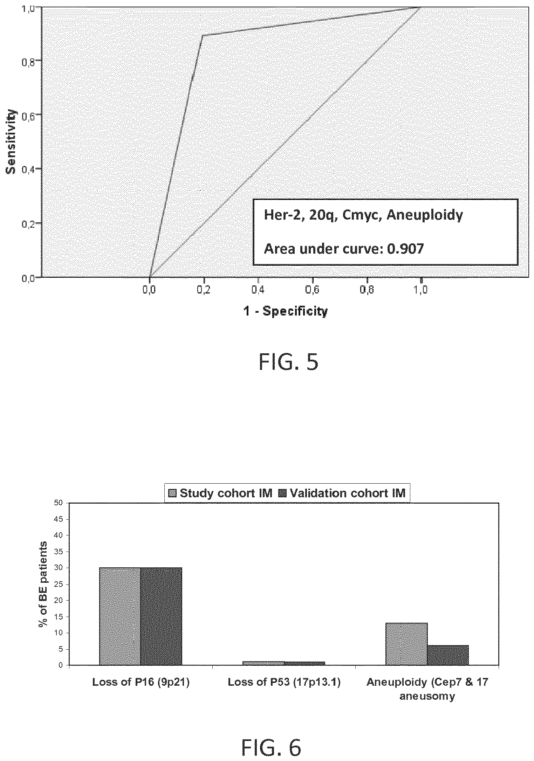

The random BE validation cohort consisted of 305 prospectively included BE patients, 266 patients with IM, 31 with LGD and 8 patients with HGD. Abnormalities for Her2/Neu (17q11.2) were observed in 62% (5/8), whereas abnormalities for c-Myc (8q24) were observed in 50% (4/8) and abnormalities for 20q were observed in 38% (3/8) in HGD cases. None of the IM or LGD cases showed these abnormalities. 75% (6/8) HGD showed aneuploidy, while aneuploidy was also seen in 5% (14/266) of ND/IND and 34% (10/29) of LGD (Fig. 4). ROC analysis showed that the combination of Her2/Neu, c-Myc, 20q13.2 and aneuploidy relate to a 0.90 area under the curve (p=0.001; sensitivity 88%, specificity 100%; Fig. 5).

**Table IV: Sensitivity and Specificity of the Diagnostic Markers in the Two Independent BE Cohorts**

| | Study Cohort (n=202) | | Validation Cohort (n=305) | |
|---|---|---|---|---|
| Marker | Sensitivity (%) | Specificity (%) | Sensitivity (%) | Specificity (%) |
| Her-2/neu | 53 | 100 | 62.5 | 100 |
| C-myc | 53 | 100 | 75 | 98 |
| 20q13.2 | 44 | 100 | 38 | 100 |
| aneuploidy (aneusomy (CEP7 and CEP 17)) | 60 | 88 | 87.5 | 91 |

### Example 4

This example shows that progression-free survival was significantly poorer for patients with p53 abnormalities detected by FISH, IHC and the combination of the techniques, but that alone FISH has the highest positive predictive (PPV) and negative predictive values (NPV) of FISH for predicting progression with BE, which can be divided into three categories: 1) low-risk patients, who have a risk of 0.2-0.5% per year to progress and mostly present with no dysplasia or indefinite dysplasia, 2) intermediate-risk patients, who have a risk of 1 to 10% per year to progress and who may present with LGD, and 3) patients, who have HGD or early cancer and demand treatment.

The prognostic markers as addressed above were tested in the low-risk population while the diagnostic markers were tested in a mixed population of Barrett patients. In this study the role of p53 was more specifically addressed. Although not mentioned in guidelines, immunohistochemistry (IHC) for p53 is currently being applied in many pathology laboratories to determine if Barrett patients are running an intermediate risk to progress. In the low-risk cohorts mentioned in examples 1 and 2, only a small fraction of non-dysplastic Barrett patients was identified (Fig. 6).

In a sub analysis p53 losses/CEP 17 status assessed by FISH on brush cytology specimens were evaluated as compared to p53 losses assessed by IHC on biopsy specimens, wherein all of the specimens were taken at t=0 in a mixed cohort of Barrett patients. Prospective surveillance was performed for patients with no/indefinite dysplasia and LGD dysplasia. In this study116 BE patients were initially evaluated, of which 95 were male and 21 were female. The mean and median ages were 63 ± 13 years and 64 years (range 34-92), respectively. Median length of the maximal BE segment (M length) was 3 cm (range 1-13 cm). The BE population included 80 intestinal-type metaplasia with no dysplasia (IM), 13 indefinite for dysplasia (IND), seven low-grade dysplasia (LGD), six high-grade dysplasia (HGD), and 10 esophageal adenocarcinoma (EAC) cases, as assessed by histopathology on biopsy specimens taken at the inclusion of the patients (t=0). FISH analysis was performed using specific probes for LSI p53 and CEP17 to determine the ratio between CEP 17 and p53. A ratio of p53:CEP17<1 was considered as (relative) p53 loss. Loss or relative loss was determined by counting matched squamous cytology controls. These squamous cytology samples were acquired at t=0 and processed as a second cytospin sample on the same slide as the Barrett specimens. FISH was performed simultaneously for the (internal) controls and Barrett cases. The cut off value was determined by the mean of the internal controls+3SD.

For IHC, 8.6% of the IM/IND cases, 100% of the LGD, 66.7% of the HGD and 80% of the EAC cases were positive for p53 protein accumulation. For FISH, 7.5% of the IM/IND cases, 42.9% of the LGD, 33.3% of the HGD and 80% of the EAC cases were positive for p53 locus loss. The data showed an increase in p53 abnormality frequency along the BE pathogenic sequence. This significant correlation is seen for both IHC and FISH (Chi²-trend, *p* < *0.001*).

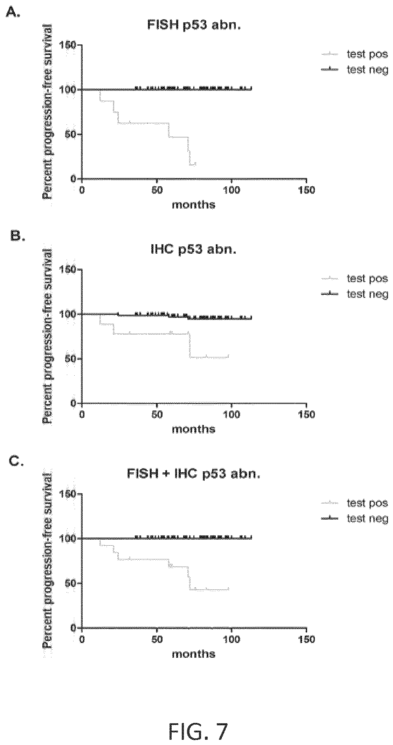

Prospective follow-up was performed for 86 of the patients with IM, IND and LGD following ACG criteria and the routine endoscopic biopsy guidelines. Mean and median ages of the follow-up cohort were 62 ± 13 yrs and 61 yrs (range 34-88), respectively. Seventy three of these patients were male and 13 were female. The follow-up cohort included 73 IM, 9 IND and 4 LGD histopathological cases. The mean follow-up time was 63 months, ranging from 1 to 113 months. Endpoints of follow-up were progression-defined as development of IM/IND to HGD/EAC and development of LGD to HGD/EAC. Patients that progressed within six months were excluded from the analysis as in such cases it is likely that the more advanced pathogenic stage was missed at the first endoscopy. Mean time to progression was 43 months, ranging from 12 to 72 months. The positive predictive (PPV) and negative predictive values (NPV) of FISH for predicting progression were 75 and 100%, respectively. For IHC the PPV and NPV were 33.3 and 96.1%. Progression-free survival for test positive and test negative groups for both techniques and the combination of the techniques were evaluated by Kaplan-Meier analysis (Fig. 7a-7c). The progression-free survival was significantly poorer for patients with p53 abnormalities detected by FISH, IHC and the combination of the techniques (log-rank test p-value was respectively <0.001, 0.004 and <0.001; Fig. 7a-7c).

All patents, patent application publications, journal articles, textbooks, and other publications mentioned in the specification are indicative of the level of skill of those in the art to which the disclosure pertains.

The invention illustratively described herein may be suitably practiced in the absence of any element(s) or limitation(s), which is/are not specifically disclosed herein. Thus, for example, each instance herein of any of the terms "comprising," "consisting essentially of," and "consisting of" may be replaced with either of the other two terms. Likewise, the singular forms "a," "an," and "the" include plural references unless the context clearly dictates otherwise. Thus, for example, references to "the method" includes one or more methods and/or steps of the type, which are described herein and/or which will become apparent to those ordinarily skilled in the art upon reading the disclosure.

The terms and expressions, which have been employed, are used as terms of description and not of limitation. In this regard, where certain terms are defined under "Definitions" and are otherwise defined, described, or discussed elsewhere in the "Detailed Description," all such definitions, descriptions, and discussions are intended to be attributed to such terms. There also is no intention in the use of such terms and expressions of excluding any equivalents of the features shown and described or portions thereof. Furthermore, while subheadings, e.g., "Definitions," are used in the "Detailed Description," such use is solely for ease of reference and is not intended to limit any disclosure made in one section to that section only; rather, any disclosure made under one subheading is intended to constitute a disclosure under each and every other subheading.

The invention regards the follow item following items:
1. A method of prognosticating progression of Barrett's esophagus in a patient, which method comprises detecting in a sample of esophageal cells from the patient at least one abnormality selected from the group consisting of p16 loss, p53 loss, chromosome 7 aneuploidy, and chromosome 17 aneuploidy, wherein the presence of at least one abnormality indicates that Barrett's esophagus will likely progress towards esophageal adenocarcinoma, whereupon progression of Barrett's esophagus in the patient is prognosticated.
2. The method of item 1, wherein the at least one abnormality is p16 loss.
3. The method of item 1, wherein the at least one abnormality is p16 loss, chromosome 7 aneuploidy, and chromosome 17 aneuploidy.
4. The method of item 1, which further comprises detecting in the sample of esophageal cells from the patient at least one abnormality selected from the group consisting of 20q gain, C-myc gain, and Her2 gain.
5. The method of item 1, wherein the sample of esophageal cells is obtained by a brush cytology method.
6. A set of probes comprising a probe for p16, a probe for p53, a probe for chromosome 7, and a probe for chromosome 17.
7. The set of probes of item 6, which further comprises at least one additional probe selected from the group consisting of a probe for 20q, a probe for c-myc, and a probe for Her2.
8. The set of probes of item 6, which further comprises a probe for 20q, a probe for c-myc, and a probe for Her2.
9. A kit comprising (a) a set of probes that enables prognosis of progression of Barrett's esophagus in a patient, wherein the set of probes comprises a probe for p16, a probe for p53, a probe for chromosome 7, and a probe for chromosome 17 and (b) instructions for prognosticating the progression of Barrett's esophagus in a patient, wherein the instructions comprise determining in a sample of esophageal cells from a patient at least one abnormality selected from the group consisting of p16 loss, p53 loss, chromosome 7 aneuploidy, and chromosome 17 aneuploidy.
10. The kit of item 9, wherein the set of probes of (a) further comprises at least one additional probe, wherein the at least one additional probe is selected from the group consisting of a probe for 20q, a probe for C-myc, and a probe for Her2, and the instructions of (b) further comprises determining in a sample of esophageal cells from a patient at least one additional abnormality selected from the group consisting of 20q gain, C-myc gain, and Her2 gain.

## Claims

1. A method of prognosticating progression of Barrett's esophagus in a patient, which method comprises:
(a) contacting a sample of esophageal cells obtained from the patient with a set of detectably labeled probes consisting of (i) a probe for p16 which hybridizes to p21 on human chromosome 9, (ii) a probe for p53 which hybridizes to p13 on human chromosome 17, (iii) a centromeric probe for chromosome 7, (iv) a centromeric probe for chromosome 17, and (v) a probe for Her2 which hybridizes to q11 on human chromosome 17 under hybridization conditions, wherein each probe is detectably labeled with a distinct fluorophore,
(b) detecting formation of a hybridization complex between each of the detectably labeled probes and a target nucleic acid sequence by assessing a signal from each of the distinct fluorophores,
(c) determining the copy numbers of the p16 gene, the p53 gene, chromosome 7, chromosome 17, and the Her2 gene by quantifying the signal from each of the distinct fluorophores,
(d) comparing the copy numbers determined in (c) to a pre-determined cut-off for each of the p16 gene, the p53 gene, chromosome 7, chromosome 17, and the Her2 gene, wherein a copy number greater than the pre-determined cut-off indicates a gain of the gene or chromosome and a copy number less than the pre-determined cut-off indicates a loss of the gene or chromosome, and wherein loss of the p16 gene, loss of the p53 gene, chromosome 7 aneuploidy, chromosome 17 aneuploidy, and/or Her2 gene amplification indicates progression of Barrett's esophagus.

2. The method of claim 1, further comprising detecting in the sample of esophageal cells a gain of chromosome 20q and/or a gain of the c-myc gene.

3. The method of claim 1, wherein the sample of esophageal cells is obtained by a brush cytology method.

## Patentansprüche

1. Ein Verfahren zur Vorhersage der Entwicklung von Barret-Ösophagus in einem Patienten, wobei das Verfahren umfasst:
(a) Inkontaktbringen einer Probe von Ösophaguszellen, die von dem Patienten erhalten werden, mit einem Satz detektierbar markierter Sonden, bestehend aus (i) einer Sonde für p16, das an p21 auf dem menschlichen Chromosom 9 hybridisiert, (ii) einer Sonde für p53, das an p13 auf dem menschlichen Chromosom 17 hybridisiert, (iii) einer zentromerischen Sonde für das Chromosom 7, (iv) einer zentromerischen Sonde für das Chromosom 17, und (v) einer Sonde für Her2, das an q11 auf dem menschlichen Chromosom 17 unter Hybridisierungsbedingungen hybridisiert, wobei jede Sonde detektierbar mit einem eindeutigen Fluorophor markiert ist,
(b) Detektieren der Bildung eines Hybridisierungskomplexes zwischen jeder der detektierbar markierten Sonden und einer Ziel-Nukleinsäure-Sequenz durch Auswerten eines Signals von jedem der eindeutigen Fluorophore,
(c) Bestimmen der Kopienzahl des p16-Gens, des p53-Gens, des Chromosoms 7, des Chromosoms 17 und des Her2-Gens durch Quantifizieren des Signals von jedem der eindeutigen Fluorophore,
(d) Vergleichen der Kopienzahlen, die in (c) bestimmt werden, mit einem vorherbestimmten Grenzwert für jedes von dem p16-Gen, dem p53-Gen, dem Chromosom 7, dem Chromosom 17 und dem Her2-Gen, wobei eine Kopienzahl größer als der vorherbestimmte Grenzwert eine Verstärkung des Gens oder Chromosoms anzeigt, und eine Kopienzahl kleiner als der vorherbestimmte Grenzwert einen Verlust des Gens oder Chromosoms anzeigt, und wobei ein Verlust des p16-Gens, ein Verlust des p53-Gens, eine Chromosom 7-Aneuploidie, eine Chromosom 17-Aneuploidie und / oder eine Her2-Genamplifikation eine Entwicklung von Barret-Ösophagus anzeigt.

2. Das Verfahren nach Anspruch 1, ferner umfassend:
Detektieren, in der Probe von Ösophaguszellen, einer Verstärkung des Chromosoms 20 und/oder einer Verstärkung des c-myc-Gens.

3. Das Verfahren nach Anspruch 1, wobei die Probe von Ösophaguszellen durch ein Bürsten-Zytologieverfahren erhalten wird.

## Revendications

1. Méthode permettant de pronostiquer la progression de l'oesophage de Barrett chez un patient, ladite méthode comprenant :
(a) la mise en contact d'un échantillon de cellules oesophagiennes prélevées sur le patient avec un ensemble de sondes marquées pour être détectables, se composant (i) d'une sonde pour p16, qui s'hybride à p21 sur le chromosome humain 9, (ii) d'une sonde pour p53, qui s'hybride à p13 sur le chromosome humain 17, (iii) d'une sonde centromérique pour le chromosome 7, (iv) d'une sonde centromérique pour le chromosome 17, et (v) d'une sonde pour Her2, qui s'hybride à q11 sur le chromosome humain 17 dans des conditions d'hybridation, où chaque sonde est marquée pour être détectable avec un fluorophore distinct,
(b) la détection de la formation d'un complexe d'hybridation entre chacune des sondes marquées pour être détectables et une séquence d'acide nucléique cible en évaluant un signal provenant de chacun des fluorophores distincts,
(c) la détermination des nombres de copies du gène p16, du gène p53, du chromosome 7, du chromosome 17, et du gène Her2 en quantifiant le signal provenant de chacun des fluorophores distincts,
(d) la comparaison des nombres de copies déterminés au (c) à une valeur de cut-off prédéfinie pour chacun des gène p16, gène p53, chromosome 7, chromosome 17, et gène Her2, où un nombre de copies supérieur à la valeur de cut-off prédéfinie indique un gain du gène ou du chromosome et un nombre de copies inférieur à la valeur de cut-off prédéfinie indique une perte du gène ou du chromosome, et où la perte du gène p16, la perte du gène p53, l'aneuploïdie du chromosome 7, l'aneuploïdie du chromosome 17, et/ou l'amplification du gène Her2 indiquent la progression de l'oesophage de Barrett.

2. Méthode selon la revendication 1, comprenant en outre la détection, dans l'échantillon de cellules oesophagiennes, d'un gain du chromosome 20q et/ou d'un gain du gène c-myc.

3. Méthode selon la revendication 1, dans laquelle l'échantillon de cellules oesophagiennes est obtenu par une méthode de cytologie à brosse.
